(19) Europäisches Patentamt
European Patent Office
Office européen des brevets

(11) **EP 2 338 819 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**24.02.2021   Patentblatt 2021/08**

(45) Hinweis auf die Patenterteilung:
**11.07.2012   Patentblatt 2012/28**

(21) Anmeldenummer: **10016119.9**

(22) Anmeldetag: **21.09.2009**

(51) Int Cl.:
**B65H 69/00** (2006.01)    **B65H 63/06** (2006.01)

(54) **Qualitätsüberwachung von Spleissen in einem länglichen textilen Prüfgut**

Monitoring the quality of splices in an elongated textile test material

Surveillance de la qualité d'épissures dans un produit de test textile longitudinal

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **29.09.2008   CH 15452008**

(43) Veröffentlichungstag der Anmeldung:
**29.06.2011   Patentblatt 2011/26**

(62) Dokumentnummer(n) der früheren Anmeldung(en) nach Art. 76 EPÜ:
**09775790.0 / 2 331 441**

(73) Patentinhaber: **Uster Technologies AG**
**8610 Uster (CH)**

(72) Erfinder:
• **Narayanan, Sivakumar**
  **8610 Uster (CH)**
• **Schmid, Peter**
  **8050 Zürich (CH)**
• **Soell, Wolfram**
  **8610 Uster (CH)**
• **Geiter, Paul**
  **8808 Pfäffikon (CH)**
• **Keller, Beat**
  **8046 Zürich (CH)**
• **Gehrig, Stefan**
  **8610 Uster (CH)**

(74) Vertreter: **Pliska, Pavel**
**Uster Technologies AG**
**Sonnenbergstrasse 10**
**8610 Uster (CH)**

(56) Entgegenhaltungen:
| | |
|---|---|
| EP-A- 1 077 194 | EP-A- 1 101 846 |
| EP-A2- 0 365 901 | EP-A2- 1 295 835 |
| WO-A-2007/056883 | DE-A1- 3 812 449 |
| DE-A1- 3 937 824 | DE-A1- 4 004 755 |
| DE-A1- 4 028 465 | DE-A1- 4 335 256 |
| DE-A1- 19 649 329 | US-A- 3 297 346 |
| US-A- 4 314 437 | US-B1- 6 374 152 |

• **Anonym: "Uster Quantum 2 Anwendungshandbuch", , vol. V1.1, 1 May 2005 (2005-05-01),**

EP 2 338 819 B2

**Beschreibung**

FACHGEBIET

Die vorliegende Erfindung liegt auf dem Gebiet der Qualitätsprüfung von Textilmaterialien. Sie betrifft eine Vorrichtung zur Überwachung von Spleissen in einem länglichen textilen Prüfgut, insbesondere Garn, gemäss dem Oberbegriff des unabhängigen Patentanspruchs. Sie eignet sich besonders gut für den Einsatz in so genannten Garnreinigern auf Spinn- oder Spulmaschinen.

STAND DER TECHNIK

Eine Vorrichtung und ein Verfahren zur Überwachung von Spleissen in einem Faden (Garn) sind aus der DE-40'28'465 A1 bekannt. Dabei ist eine Einrichtung zum Messen der Dicke und/oder Masse des Fadens und seiner im Fadenverbindungselement (Spleisser) herzustellenden Verbindungsstelle (Spleiss) angeordnet. Die Einrichtung steht in Wirkverbindung mit einer Fadentrenneinrichtung. Die Fadentrenneinrichtung wird durch die Einrichtung gesteuert. Diese Einrichtung ist so ausgebildet, dass die Fadentrenneinrichtung dann aktiviert wird, wenn eine Verbindungsstelle ausserhalb einer nicht weiter bezeichneten Toleranz liegt.

Die DE-39'37'824 A1 offenbart eine Spulstelle einer automatischen Spulmaschine. Im Fadenpfad der Spulstelle ist ein elektronischer Fadenreiniger eingebaut, der Dickstellen, Dünnstellen und Doppelfäden erkennt. Der Fadenreiniger prüft auch frisch erstellte Spleissverbindungen darauf hin, ob sie in einem vorgegebenen Toleranzbereich liegen. Wie dieser Toleranzbereich definiert ist, wird nicht ausgeführt.

Die DE-196'49'329 A1 beschreibt ein Verfahren zum Überprüfen des Fadenprofils an einem laufenden Faden beim Anspinnen in einer Offenend-Spinnmaschine. Es wird festgestellt, dass die Darstellung des Fadenquerschnitts des Anspinners über seine Länge in der Regel eine M-förmige Kurve ergibt. Ausgehend von dieser Feststellung wird der Anspinner in drei Bereiche unterteilt. Ein fehlerhafter Anspinner liegt vor, wenn in einem den beiden am Rand des Anspinners liegenden Bereiche der gemessene Fadenquerschnitt einen vordefinierte Grenzwert überschreitet.

Die EP-1'101'846 A2 befasst sich mit der Ermittlung von Parametern eines automatischen Anspinnvorgangs. Sie zeigt eine Spinnstelle einer Offenend-Rotorspinnmaschine, in deren Fadenpfad eine Sensoreinrichtung das Fadenprofil überprüft. Die Überprüfung des Fadenprofils erfolgt am beschleunigten Faden. Die Messfrequenz der Sensoreinrichtung wird auf die sich ändernde Geschwindigkeit des beschleunigten Fadens eingestellt, indem sie an die Drehgeschwindigkeit der Fadenabzugswalze angepasst wird. Die Spinnstelle ist mit einer Einrichtung zur Visualisierung des Anspinnerprofils ausgestattet.

Aus der EP-1'077'194 A2 ist eine Spulstelle mit einer Spleisseinrichtung bekannt, bei der Flüssigkeit in die Spleissverbindung eingebracht wird, um die Festigkeit der Spleissverbindung zu verbessern. In einem kapazitiven Sensor wird die Spleissverbindung erfasst, um die Wassermenge im Spleiss zu bestimmen und gegebenenfalls zu optimieren, um die gewünschte Festigkeit der Spleissverbindung sicherzustellen.

Ein Verfahren zum Einstellen einer Reinigungsgrenze bei einem elektronischen Garnreiniger ist aus der EP-1'295'835 A2 bekannt. Dabei werden die möglichen Garnfehler in einem Sortierschema, sortiert nach Fehlerwert und Fehlerlänge, angeordnet. Es wird eine Schar von möglichen Reinigungskurven in einer Speichereinrichtung zum Editieren bereitgehalten. Daraus wird eine Reinigungskurve ausgewählt und durch Verschieben genau eines Einstellpunktes im Sortierschema festgelegt.

Aus der US-6,374,152 B1 ist ferner ein Verfahren zur Bestimmung des Verlaufes einer Reinigungsgrenze für Fehler in einem Garn bekannt. Dabei werden Parameter der Fehler in einem Diagramm aufgezeichnet und daraus eine Fehlerdichte bestimmt, die beispielsweise bei Konzentrationen von Fehlern in einem Bereich des Diagramms entsprechend hoch ausfällt. Die Reinigungsgrenze für die Fehler im Garn wird so gelegt, dass sie hohe Konzentrationen von Fehlern umgeht, so dass häufige Fehler nicht ausgereinigt werden.

Die EP-1'249'422 A2 offenbart eine Garnreinigungseinrichtung an der Spulstelle einer Textilmaschine. Die Garnreinigungseinrichtung überwacht laufend die Querdimension des Garns. Zusätzlich misst sie die Längsausdehnung von Garnfehlern, indem die Querdimension des Garns an zwei in Bewegungsrichtung des Garns hintereinander angeordneten Messpunkten gemessen und die Messungen mit einem Laufzeitkorrelator ausgewertet werden.

Weiter ist aus der WO-2007/056883 A2 ein Verfahren zur Charakterisierung von Effektgarnen bekannt, mit dem die Effekte beispielsweise in einem Diagramm mit Parametern wie Effektlänge und Massenzunahme dargestellt werden können.

Ein Nachteil dieser bekannten Verfahren ist darin zu sehen, dass sie keine differenzierten Kriterien für die Ausscheidung von Spleissen angeben. Eine Beurteilung der Spleisse erfolgt, falls überhaupt, nur hinsichtlich ihres Durchmessers oder ihrer Masse bzw. nur hinsichtlich ihrer Festigkeit über den Wassergehalt.

DARSTELLUNG DER ERFINDUNG

Es ist deshalb eine Aufgabe der vorliegenden Erfindung, eine Vorrichtung zur Überwachung der Qualität eines länglichen, in seiner Längsrichtung durch einen elektronischen Messkopf bewegten textilen Prüfgutes anzugeben, welche den obigen Nachteil vermeidet. Die Qualität der Spleisse, besonderes was ihre visuelle Erscheinung betrifft, und somit auch die Qualität des betreffenden Garns sollen verbessert werden. Schlecht funktionierende Spleisseinheiten sollen identifiziert werden, damit sie neu eingestellt, repariert oder ersetzt werden können. Zudem sollen die Vorrichtungen einfach bedienbar sein.

Diese und andere Aufgaben werden durch die Erfindung gelöst, wie sie im unabhängigen Patentanspruch definiert ist. Vorteilhafte Ausführungsformen sind in den abhängigen Patentansprüchen angegeben.

Die erfindungsgemässe Vorrichtung zur Überwachung von Spleissen in einem länglichen textilen Prüfgut gemäss der Erfindung beinhaltet einen elektronischen Messkopf, durch welchen das Prüfgut in seiner Längsrichtung bewegbar ist, zur Messung mindestens eines Parameters des Prüfgutes. Die Vorrichtung beinhaltet auch eine Steuereinheit zum Einteilen von Spleissen aufgrund mindestens eines gemessenen Parameters in zulässige bzw. unzulässige Spleisse. Die Steuereinheit ist zum Verknüpfen von mindestens zwei der drei Grössen aus der Menge, die aus

> der Anzahl der unzulässigen Spleisse,
> der Anzahl der zulässigen Spleisse und
> der Anzahl der insgesamt erfassten Spleisse

besteht, zu einer einzigen, die Spleissqualität charakterisierenden Spleissqualitätsgrösse eingerichtet. Die Steuereinheit ist zur Bildung eines Verhältnisses als Verknüpfung eingerichtet, insbesondere eines Verhältnisses zwischen der Anzahl der unzulässigen Spleisse oder der Anzahl der zulässigen Spleisse einerseits und der Gesamtzahl der erfassten Spleisse andererseits. Die Steuereinheit ist ferner zur Auslösung einer Handlung, bspw. zur Ausgabe eines Alarmsignals, wenn die Spleissqualitätsgrösse einen vorgegebenen Schwellwert über- bzw. unterschreitet, eingerichtet.

Vorzugsweise wird eine von mindestens zwei Parametern des Prüfgutes abhängige Reinigungsgrenze zwischen zulässigen und unzulässigen Spleissen im Prüfgut bestimmt. Diese Reinigungsgrenze für Spleisse ist von der bekannten Reinigungsgrenze zwischen zulässigen und unzulässigen Fehlstellen im Prüfgut verschieden. Sie legt differenzierte Kriterien für die Ausscheidung von Spleissen fest. Sie liegt vorzugsweise unterhalb der ersten Reinigungsgrenze, damit die Spleisse weniger störend sind als die Fehlstellen, die sie ersetzen.

In einem nicht erfindungsgemässen Verfahren zur Überwachung der Qualität eines länglichen, in seiner Längsrichtung durch einen elektronischen Messkopf bewegten textilen Prüfgutes wird mindestens ein Parameter des Prüfgutes gemessen. Spleisse werden aufgrund mindestens eines gemessenen Parameters in zulässige bzw. unzulässige Spleisse eingeteilt. Mindestens zwei der drei Grössen aus der Menge, die aus

> der Anzahl der unzulässigen Spleisse,
> der Anzahl der zulässigen Spleisse und
> der Anzahl der insgesamt erfassten Spleisse

besteht, werden miteinander zu einer einzigen, die Spleissqualität charakterisierenden Spleissqualitätsgrösse verknüpft. Die Verknüpfung beinhaltet die Bildung eines Verhältnisses, insbesondere eines Verhältnisses zwischen der Anzahl der unzulässigen Spleisse und/oder der Anzahl zulässigen Spleisse einerseits und der Gesamtzahl der erfassten Spleisse andererseits. Es wird eine Handlung ausgelöst, bspw. ein Alarmsignal ausgegeben, wenn die Spleissqualitätsgrösse einen vorgegebenen Schwellwert über- bzw. unterschreitet. Die genannten Anzahlen beziehen sich vorzugsweise auf eine bestimmte Garnlänge, die nicht notwendigerweise bekannt sein muss. Alternativ können sie sich auf eine bestimmte Zeitdauer beziehen, die ebenfalls nicht notwendigerweise bekannt sein muss.

Wenn wir mit

> $J_u$ die Anzahl unzulässiger Spleisse,
> $J_z$ die Anzahl zulässiger Spleisse und
> $J_{tot} = J_u + J_z$ die Anzahl der insgesamt erfassten Spleisse

bezeichnen, so ergeben sich mit diesen drei Grössen die folgenden sechs Variationen zur Bildung von Quotienten:

$J_u/J_z$, $J_u/J_{tot}$, $J_z/J_{tot}$, $J_z/J_u$, $J_{tot}/J_u$, $J_{tot}/J_z$.

Jeder dieser Quotienten lässt sich durch jeden anderen ausdrücken. Irgendeiner dieser Quotienten genügt also zur Angabe einer die Spleissqualität charakterisierenden Spleissqualitätsgrösse; die anderen enthalten keine zusätzliche Information. Ein Quotient, welcher besonders anschaulich ist, ist das "Spleissverhältnis"

$$Q = J_u/J_{tot} \ ,$$

welches angibt, welcher Bruchteil der insgesamt erfassten Spleisse unzulässig ist. Dieses Spleissverhältnis Q lässt sich für jede einzelne Spleisseinheit bilden. Aus dem Spleissverhältnis kann man ableiten, ob eine bestimmte Spleisseinheit gut oder schlecht arbeitet. Dazu kann z. B. ein Schwellwert oder Grenzwert vorgegeben werden, der bspw. durch das Spleissverhältnis unterschritten werden sollte, damit die Spleisseinheit als genügend beurteilt wird. Wird der Grenzwert überschritten, so kann daraus ein Alarmsignal gewonnen werden, das bewirkt, dass eine bestimmte Spulstelle oder eine Spleisseinheit stillgesetzt wird, um sie zu überprüfen. Das Spleissverhältnis kann dauernd berechnet und sein Verlauf mit dem Grenzwert auch laufend verglichen werden.

Beim Einstellen von Reinigungsgrenzen für ein längliches textiles Prüfgut an einem elektronischen Messkopf wird eine von mindestens zwei Parametern des Prüfgutes abhängige erste Reinigungsgrenze zwischen zulässigen und unzulässigen Fehlstellen im Prüfgut festgelegt, in einem Diagramm als erste Reinigungskurve dargestellt und an den Messkopf übermittelt. Ferner wird eine von den mindestens zwei Parametern des Prüfgutes abhängige, von der ersten Reinigungsgrenze verschiedene zweite Reinigungsgrenze zwischen zulässigen und unzulässigen Spleissen im Prüfgut automatisch aus der ersten Reinigungsgrenze berechnet, in einem Diagramm als zweite Reinigungskurve dargestellt und an den Messkopf übermittelt.

Die zweite Reinigungsgrenze kann durch eine Koordinatentransformation eines durch die mindestens zwei Parameter aufgespannten Koordinatensystems, vorzugsweise durch eine Verschiebung (Translation), eine Drehung (Rotation), eine Massstabsveränderung (Skalierung) und/oder eine Scherung, aus der ersten Reinigungsgrenze hervorgehen. Die zweite Reinigungsgrenze kann durch eine Eingabe einer Bedienungsperson nachträglich verändert werden. Vorzugsweise werden die erste und die zweite Reinigungskurve in ein und demselben Diagramm dargestellt. In einer vorteilhaften Ausführungsform wird mindestens eines der Diagramme in einem zweidimensionalen kartesischen Koordinatensystem gezeichnet. Einer der mindestens zwei Parameter ist z. B. ein Mass für eine Länge der Fehlstelle bzw. des Spleisses und ein anderer der mindestens zwei Parameter ist z. B. ein Mass für eine Masse pro Längeneinheit oder eine Querdimension des Prüfgutes. Es ist von Vorteil, wenn die erste Reinigungskurve und die zweite Reinigungskurve durch mindestens ein graphisches Merkmal, z. B. eine Linienform und/oder eine Farbe, voneinander unterschieden werden.

Im Verfahren zur Überwachung der Qualität eines länglichen, in seiner Längsrichtung durch einen elektronischen Messkopf bewegten textilen Prüfgutes werden vorzugsweise eine erste und zweite Reinigungsgrenze an dem Messkopf wie oben beschrieben eingestellt. Mindestens jeweils zwei Parameter des Prüfgutes für Fehlstellen und für Spleisse im Prüfgut werden gemessen und mit der ersten bzw. zweiten Reinigungsgrenze verglichen.

Es ist vorteilhaft, wenn bei Überschreiten der zweiten Reinigungsgrenze eine Handlung ausgelöst wird, die bspw. ein Entfernen des entsprechenden Spleisses und ein Herstellen eines neuen Spleisses beinhaltet. Der neue Spleiss kann wiederum wie oben beschrieben überprüft werden. Die an dem Prüfgut gemessenen Parameter werden vorzugsweise in Form von Punktewolken (englisch: scatter plot), in denen jeder Punkt einer Fehlstelle bzw. einem Spleiss entspricht, in mindestens einem Diagramm dargestellt. Dabei können die den Fehlstellen entsprechenden Punkte einerseits und die den Spleissen entsprechenden Punkte andererseits durch mindestens ein graphisches Merkmal, z. B. eine Form und/oder eine Farbe, voneinander unterschieden werden. Ebenso können die den zulässigen Fehlstellen bzw. Spleissen entsprechenden Punkte einerseits und die den unzulässigen Fehlstellen bzw. Spleissen entsprechenden Punkte andererseits durch mindestens ein graphisches Merkmal, z. B. eine Form und/oder eine Farbe, voneinander unterschieden werden. Auch die erste Reinigungskurve und die zweite Reinigungskurve können durch mindestens ein graphisches Merkmal, z. B. eine Linienform und/oder eine Farbe, voneinander unterschieden werden.

Die zweite Reinigungsgrenze kann in Abhängigkeit von den gemessenen mindestens zwei Parametern zeitlich geändert werden. Dabei kann man analog zur US-6,374,152 B1 vorgehen. Für Bereiche des durch die Parameter aufgespannten Raums kann eine Spleissdichte bestimmt werden. Die zweite Reinigungsgrenze für die Spleisse kann so geändert werden, dass sie Bereiche mit hohen Spleissdichten umgeht, so dass nur einzelne, als Ausreisser anzusehende Spleisse ausgereinigt werden, die typischen und häufigen Spleisse jedoch im länglichen textilen Prüfgut verbleiben. Dadurch wird ein zu häufiges Ausreinigen von Spleissen vermieden und die Wirtschaftlichkeit der Vorrichtung erhöht.

Die Vorrichtung zur Überwachung von Spleissen in einem länglichen textilen Prüfgut gemäss der Erfindung beinhaltet einen elektronischen Messkopf, durch welchen das Prüfgut in seiner Längsrichtung bewegbar ist, zur Messung von mindestens jeweils zwei Parametern des Prüfgutes für Fehlstellen und für Spleisse im Prüfgut. Ferner beinhaltet die Vorrichtung vorzugsweise eine Steuereinheit, die zum Speichern einer von den mindestens zwei Parametern abhängigen ersten Reinigungsgrenze zwischen zulässigen und unzulässigen Fehlstellen im Prüfgut und zum Übermitteln der ersten Reinigungsgrenze an den Messkopf eingerichtet ist. Ausserdem beinhaltet die Vorrichtung vorzugsweise eine mit der Steuereinheit verbundene Anzeigeeinheit, die zum Darstellen der ersten Reinigungsgrenze als erste Reinigungskurve in einem Diagramm eingerichtet ist. Die Steuereinheit ist dazu eingerichtet, eine von den mindestens zwei Parametern abhängige, von der ersten Reinigungsgrenze verschiedene zweite Reinigungsgrenze zwischen zulässigen und unzulässigen Spleissen im Prüfgut aus der ersten Reinigungsgrenze automatisch zu berechnen, zu speichern und an den Messkopf zu übermitteln. Die Anzeigeeinheit ist zum Darstellen der zweiten Reinigungsgrenze als zweite Reinigungskurve in einem Diagramm eingerichtet.

In einer bevorzugten Ausführungsform beinhaltet die Vorrichtung eine mit der Auswerteeinheit verbundene Schneideinheit zum Entfernen von unzulässigen Spleissen aus dem Prüfgut.

Im Verfahren zur Überwachung der Qualität eines länglichen, in seiner Längsrichtung durch einen elektronischen Messkopf bewegten textilen Prüfgutes wird vorzugsweise eine von mindestens zwei Parametern des Prüfgutes abhängige erste Reinigungsgrenze zwischen zulässigen und unzulässigen Fehlstellen im Prüfgut festgelegt, in einem Diagramm als erste Reinigungskurve dargestellt und an den Messkopf übermittelt. Die mindestens zwei Parameter des Prüfgutes für Fehlstellen im Prüfgut werden gemessen, in Form einer ersten Punktewolke, in der jeder Punkt einer Fehlstelle entspricht, in dem Diagramm dargestellt und mit der ersten Reinigungsgrenze verglichen. Eine von den mindestens zwei Parametern des Prüfgutes abhängige zweite Reinigungsgrenze zwischen zulässigen und unzulässigen Spleissen im Prüfgut wird festgelegt, in dem Diagramm als zweite Reinigungskurve dargestellt und an den Messkopf übermittelt. Die mindestens zwei Parameter des Prüfgutes für Spleisse im Prüfgut werden gemessen, in Form einer Punktewolke, in der jeder Punkt einem Spleiss entspricht, in dem Diagramm dargestellt und mit der zweiten Reinigungsgrenze verglichen.

Die zweite Reinigungsgrenze kann frei von einer Bedienungsperson festgelegt werden. Dies wird durch die Darstellung der Spleissereignisse, der ersten Reinigungskurve und der zweiten Reinigungskurve in ein und demselben Diagramm stark erleichtert oder überhaupt erst ermöglicht. Dank der Darstellung der Spleissereignisse kann die Bedienungsperson auf einfache Weise und intuitiv die zweite Reinigungsgrenze so festlegen, dass einerseits wenig störende Spleisse nicht unnötig aus dem Garn entfernt werden, andererseits aber störende Spleisse tatsächlich entfernt werden. Die Darstellung der ersten Reinigungskurve erlaubt es der Bedienungsperson, sich bei der Festlegung der zweiten Reinigungskurve nach der ersten Reinigungskurve zu orientieren, bspw. einen genügend grossen Abstand von ihr einzuhalten. Vorzugsweise wird die Darstellung auf einem Bildschirm angezeigt, und die Festlegung der zweiten Reinigungsgrenze erfolgt durch bekannte Eingabemittel wie eine Computermaus, eine Tastatur oder einen Sensorbildschirm (touchscreen). Die zweite Reinigungskurve kann dabei frei gezeichnet oder aus einem bestehenden Satz von Kurven ausgewählt werden; danach kann sie verschoben, rotiert, gestreckt und/oder auf andere Weise transformiert werden, bis sie den gewünschten Verlauf aufweist. Dieses Vorgehen kann ähnlich sein wie dasjenige, das in der EP-1'295'835 A2 für Fehlstellen beschrieben ist.

Alternativ ist es möglich, die zweite Reinigungsgrenze automatisch einzustellen oder zumindest automatisch einen Vorschlag für den Verlauf der zweiten Reinigungsgrenze erstellen zu lassen, der dann von der Bedienungsperson noch angepasst werden kann. Die automatische Berechnung der zweiten Reinigungsgrenze für Spleisse kann z. B. auf früheren Messungen von Spleissparametern und/oder auf einer bereits existierenden ersten Reinigungsgrenze für Fehlstellen im Garn basieren.

In einer bevorzugten Ausführungsform des Verfahrens werden die den Fehlstellen entsprechenden Punkte der ersten Punktewolke einerseits und die den Spleissen entsprechenden Punkte der zweiten Punktewolke andererseits durch mindestens ein graphisches Merkmal, z. B. eine Form und/oder eine Farbe, voneinander unterschieden. Ebenso können die den zulässigen Fehlstellen bzw. Spleissen entsprechenden Punkte einerseits und die den unzulässigen Fehlstellen bzw. Spleissen entsprechenden Punkte andererseits durch mindestens ein graphisches Merkmal, z. B. eine Form und/oder eine Farbe, voneinander unterschieden werden. Bei Überschreiten der zweiten Reinigungsgrenze wird mit Vorteil eine Handlung ausgelöst, die bspw. ein Entfernen des entsprechenden Spleisses und ein Herstellen eines neuen Spleisses beinhaltet.

Die Vorrichtung zur Überwachung von Spleissen in einem länglichen textilen Prüfgut gemäss der Erfindung beinhaltet einen elektronischen Messkopf, durch welchen das Prüfgut in seiner Längsrichtung bewegbar ist, zur Messung von mindestens jeweils zwei Parametern des Prüfgutes für Fehlstellen und für Spleisse im Prüfgut. Ferner beinhaltet die Vorrichtung vorzugsweise eine Steuereinheit, die zum Speichern einer von den mindestens zwei Parametern abhängigen ersten Reinigungsgrenze zwischen zulässigen und unzulässigen Fehlstellen im Prüfgut, zum Übermitteln der ersten Reinigungsgrenze an den Messkopf und zum Vergleichen der mindestens zwei Parameter des Prüfgutes für Fehlstellen im Prüfgut mit der ersten Reinigungsgrenze eingerichtet ist. Die Vorrichtung beinhaltet vorzugsweise auch eine mit der Steuereinheit verbundene Anzeigeeinheit, die zum Darstellen der ersten Reinigungsgrenze als erste Reinigungskurve in einem Diagramm und zum Darstellen er mindestens zwei Parameter des Prüfgutes für Fehlstellen im Prüfgut in Form einer ersten Punktewolke, in der jeder Punkt einer Fehlstelle entspricht, in dem Diagramm eingerichtet ist. Die Steuereinheit ist dazu eingerichtet, eine von den mindestens zwei Parametern abhängige zweite Reinigungsgrenze zwischen zulässigen und unzulässigen Spleissen im Prüfgut zu speichern und an den Messkopf zu übermitteln.

Die Anzeigeeinheit ist zum Darstellen der zweiten Reinigungsgrenze als zweite Reinigungskurve in einem Diagramm sowie zum Darstellen der mindestens zwei Parameter des Prüfgutes für Spleisse im Prüfgut in Form einer Punktewolke, in der jeder Punkt einem Spleiss entspricht, in dem Diagramm eingerichtet.

Dank der Erfindung kann die Qualität der Spleisse dahingehend verbessert werden, dass die Spleisse in einem länglichen textilen Prüfgut, das später möglicherweise zu einem Gewebe oder Gewirke verarbeitet wird, weniger auffallen und als Fehler erscheinen. Somit wird auch die Qualität des länglichen textilen Prüfgutes weiter verbessert. Das Verfahren erlaubt es auch, die Textilmaschinen effizient zu betreiben, indem schlecht funktionierende Spleisseinheiten erkannt und repariert werden können. Dadurch wird die Produktivität der Textilmaschinen erhöht.

AUFZÄHLUNG DER ZEICHNUNGEN

Nachfolgend wird die Erfindung anhand der Zeichnungen detailliert erläutert.

Figur 1      zeigt schematisch eine Spleissverbindung in einem Garn.

Figur 2      zeigt ein Diagramm mit einer Darstellung von Spleissverbindungen anhand von Parametern in einer Punktewolke.

Figur 3      zeigt schematisch drei Verfahren, um eine Reinigungsgrenze für Spleisse aus einer Reinigungsgrenze für Fehlstellen zu berechnen.

Figuren 4-6  zeigen Diagramme mit Darstellungen von Fehlstellen und Spleissverbindungen anhand von Parametern.

Figur 7      zeigt eine Vorrichtung zur Durchführung des Verfahrens.

Figur 8      zeigt einen Geschwindigkeitsverlauf eines Garns beim Spleissen.

Figur 9      zeigt ein Zustandsdiagramm für die erfindungsgemässe Vorrichtung.

Figur 10     zeigt eine grafische Darstellung des Spleissverhältnisses in Abhängigkeit von der Zeit für eine Spleisseinheit.

Figur 11     zeigt eine Darstellung von Spleissverhältnissen für mehrere Spleisseinheiten.

AUSFÜHRUNG DER ERFINDUNG

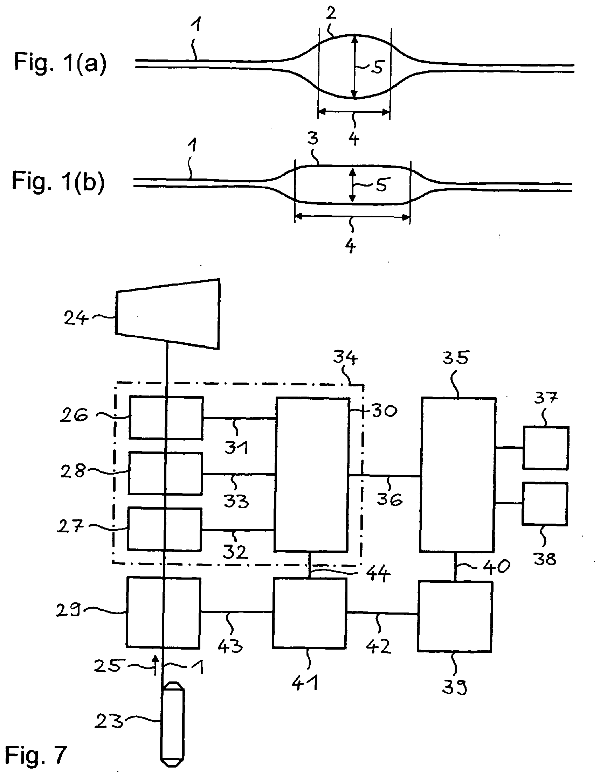

**Figur 1(a)** zeigt eine Fehlstelle 2 in einem länglichen textilen Prüfgut 1, bspw. einem Garn. Derartige Fehlstellen 2 können z. B. Dickstellen (wie im vorliegenden Beispiel), Dünnstellen oder Fremdstoffe sein. Ihre Entstehung, Überwachung, Klassierung, Beurteilung und Entfernung aus dem Garn 1 ist im Stand der Technik vielfach beschrieben und braucht hier nicht weiter diskutiert zu werden. **Figur 1(b)** zeigt in analoger Weise einen Spleiss 3 in einem länglichen textilen Prüfgut 1, bspw. einem Garn.

Für die Fehlstelle 2 bzw. den Spleiss 3 ist ein Parameter 5 dargestellt, der dem Durchmesser der Fehlstelle 2 bzw. des Spleisses 3 entspricht oder der ein Mass für die Masse pro Längeneinheit der Fehlstelle 2 bzw. des Spleisses 3 ist. Für die Fehlstelle 2 bzw. den Spleiss 3 soll auch ein weiterer Parameter wie ihre bzw. seine Länge 4 definiert sein. Die Fehlstellen- bzw. Spleisslänge 4 kann als Länge zwischen gewissen wählbaren Grenzdurchmessern in einem Übergangsbereich zwischen dem Garn 1 und der Fehlstelle 2 bzw. dem Spleiss 3 gemessen werden; zu Beginn der Fehlstelle 2 bzw. des Spleisses 3 wird ein Grenzdurchmesser längs des Garns 1 überschritten und am Ende der Fehlstelle 2 bzw. des Spleisses 3 wird ein Grenzdurchmesser längs des Garns unterschritten. Solche Konventionen zur Definition der Länge 4 eines Abschnitts eines Garns 1 sind für Fehlstellen 2 in Garn 1 oder für Effekte in Effektgarn (vgl. WO-2007/056883 A2) bereits bekannt und auch für Spleisse 3 anwendbar. Zur Messung der Fehlstellen- bzw. Spleisslänge 4 können an sich bekannte Verfahren angewendet werden, die z. B. die Messung der Momentangeschwindigkeit des Garns 1 und die Ermittlung der Länge 4 eines Garnabschnitts durch Integration der Momentangeschwindigkeiten über einen bestimmten Zeitabschnitt. Die Momentangeschwindigkeit kann aus einem Nutentrommelsignal oder aus einem Laufzeitkorrelationsverfahren, wie es die EP-1'249'422 A2 offenbart, erhalten werden.

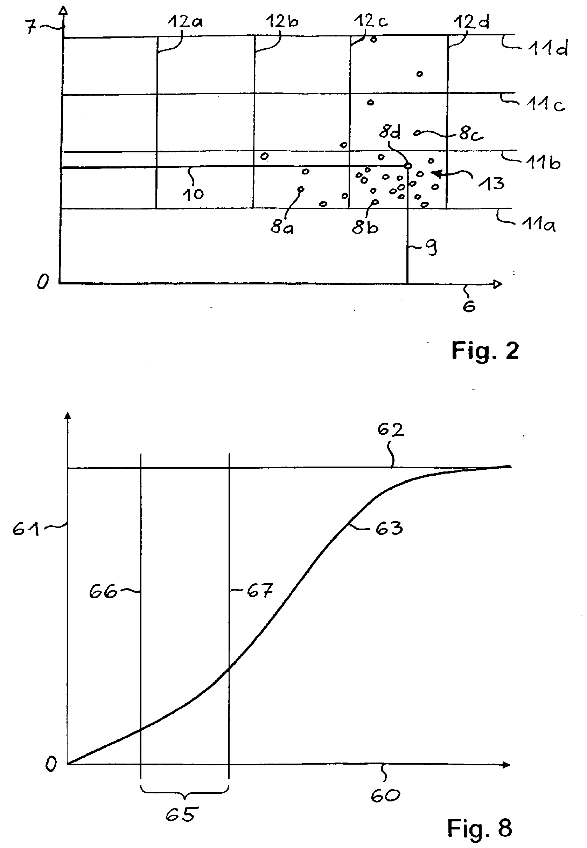

**Figur 2** zeigt ein Diagramm, wie es bereits für die Darstellung von Fehlstellen 2 in einem Garn 1 bekannt ist. Längs einer Abszisse 6 können als Parameter Werte für Längen 4 (vgl. Figur 1) und längs einer Ordinate 7 können als Parameter Werte für Durchmesser 5 (vgl. Figur 1) oder Massen pro Längeneinheit oder für die entsprechenden Änderungen von Durchmesser 5 oder Masse aufgetragen sein. Dabei entspricht der Wert null auf der Parameterachse 7 einem Sollwert, z. B. einem Solldurchmesser des Garns 1; alternativ könnte der Sollwert mit 100 % bezeichnet werden. Punkte 8a, 8b, 8c, 8d usw. bezeichnen Spleisse 3 (vgl. Figur 1(b)) oder Spleissereignisse, die durch ihre entsprechenden Parameterwerte 4, 5 auf der Abszisse 6 bzw. der Ordinate 7 gekennzeichnet sind. Die Spleissereignisse sind also im Diagramm von Figur 2 als Punktewolke 13 dargestellt. Geraden 9 und 10 bzw. ihre Schnittpunkte mit der Abszisse 6 und der Ordinate 7 verdeutlichen hier nur für den Punkt 8d, wie die Koordinaten 6, 7 einen einem Spleiss 3 zugeordneten Punkt 8d definieren. Weitere waagrechte Geraden 11a bis 11d und senkrechte Geraden 12a bis 12d bilden zusammen Rechtecke, die als Klassen bekannt sind, wie sie für die Klassierung von Fehlstellen 2 in Garnen 1 bereits eingeführt sind. Die Geraden 11 und 12 bilden somit Klassengrenzen für Spleissereignisse 8a-8d. Ein solches Diagramm stellt deshalb ein Klassierfeld für die Spleissereignisse 8a-8d dar. Die Punkte 8a-8d und weitere hier eingezeichnete Punkte bilden eine Punktewolke 13 im Klassierfeld. Für Spleisse 3 liegen typische Längen 4 zwischen ca. 2 und 10 cm, typische Durchmesser 5 bzw. Massen pro Längeneinheit zwischen ca. 150 und 200 % des Solldurchmessers bzw. der Sollmasse des Garns 1.

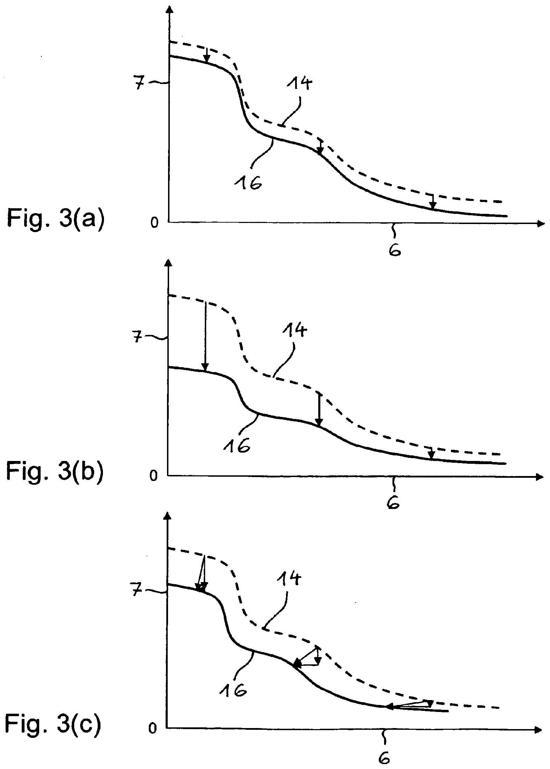

**Figur 3** zeigt Diagramme mit Achsen 6, 7, wie bereits für die Figur 2 beschrieben. Man erkennt hier insbesondere eine erste so genannte Reinigungskurve 14 für Fehlstellen oder Fehler 2 (siehe Figur 1(a)) und eine zweite Reinigungskurve 16 für Spleisse 3 (siehe Figur 1(b)) im Garn 1. Die Reinigungskurven 14, 16 sind graphische Darstellungen von entsprechenden Reinigungsgrenzen für Fehlstellen 2 bzw. Spleisse 3. Die Reinigungsgrenzen sind Beurteilungskriterien für die Qualität von Fehlstellen 2 bzw. Spleissen 3. Falls der Ereignispunkt 8 (vgl. Figur 2) einer Fehlstelle 2 unterhalb der ersten Reinigungsgrenze 14 liegt, ist die Fehlstelle 2 zulässig, sonst unzulässig. Analoges gilt für Spleisse 3. In den drei Diagrammen von Figur 3 sind nun drei verschiedene Verfahren beschrieben, um die zweite Reinigungsgrenze automatisch aus der ersten, vorgegebenen Reinigungsgrenze zu berechnen. Bei allen drei Ausführungsformen handelt es sich um affine Koordinatentransformationen. In den Diagrammen ist jeweils mit Pfeilen angedeutet, wie drei zufällig gewählte Punkte auf der ersten Reinigungskurve 14 in die drei ihnen zugeordneten Punkte auf der zweiten Reinigungskurve 16 übergehen.

Gemäss der Ausführungsform von **Figur 3(a)** entsteht die zweite Reinigungskurve 16 durch eine Verschiebung (Translation) der ersten Reinigungskurve 14 parallel zur Ordinate 7. Ein Punkt (L, D) auf der ersten Reinigungskurve 14 geht also in einen Punkt (L', D') auf der zweiten Reinigungskurve 16 über; es gilt: L' = L, D' = D - b, wobei (0,-b) ein Verschiebungsvektor ist.

Im Ausführungsbeispiel von **Figur 3(b)** geht die zweite Reinigungskurve 16 durch Stauchung der ersten Reinigungskurve 14 in Richtung der Ordinate 7, was einen Spezialfall der Massstabsveränderung (Skalierung) darstellt. Der Ordinatenwert eines jeden Punktes auf der ersten Reinigungskurve 14 wird mit einem Skalierungsfaktor, z. B. 0.6, multipliziert, um den Ordinatenwert des entsprechenden Punktes auf der zweiten Reinigungskurve 16 zu erhalten. Es gilt: L' = L, D' = 0.6·D. Dies ergibt eine Stauchung um 40 % in Richtung der Ordinate 7.

**Figur 3(c)** zeigt einen allgemeineren Fall der Skalierung, in dem sowohl in Richtung der Ordinate 7 als auch der Abszisse 6 gestaucht wird, bspw. jeweils mit dem Skalierungsfaktor 0.8. Es gilt: L' = 08·L, D' = 0.8·D. Die Skalierungsfaktoren für die beiden Richtungen brauchen selbstverständlich nicht dieselben zu sein. Das Fehlen einer zweiten Reinigungskurve 16 für sehr lange Spleisse 3 stellt in der Praxis kein Problem dar, weil derart lange Spleisse ohnehin kaum vorkommen. Bei Bedarf kann jedoch die zweite Reinigungskurve 16 für grosse Werte auf der Abszisse 6 in geeigneter Weise verlängert werden.

Der Fachmann ist bei Kenntnis der Erfindung in der Lage, weitere Ausführungsbeispiele für eine Berechnung der zweiten Reinigungsgrenze aus der ersten Reinigungsgrenze anzugeben. Es kann sich um affine Koordinatentransformationen wie Verschiebung (Translation), Drehung (Rotation), Massstabsveränderung (Skalierung), Scherung oder um Kombinationen derselben handeln. Ferner sind viele andere Funktionen denkbar, welche jedem Punkt auf der ersten Reinigungskurve 14 einen Punkt auf der zweiten Reinigungskurve 16 zuordnen.

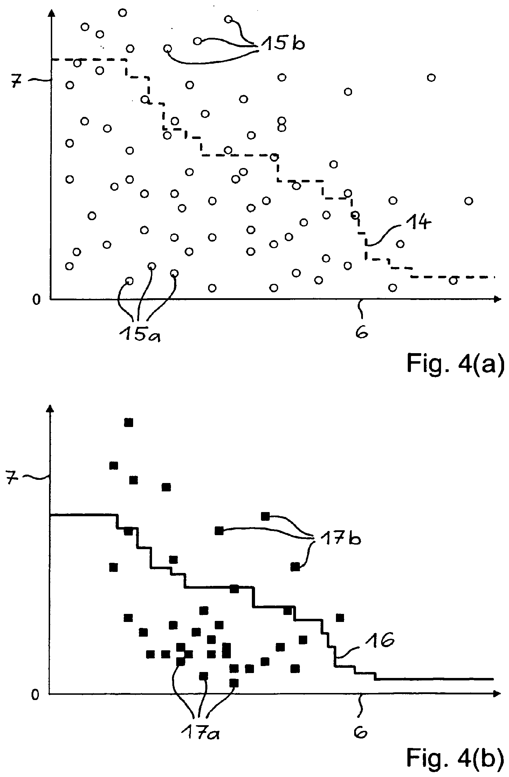

Auch in **Figur 4** sind die bekannten Diagramme mit Achsen 6, 7, dargestellt. Im Diagramm von **Figur 4(a)** sind Punkte 15a, 15b eingezeichnet, die Fehlerpunkte oder Fehlerereignisse 15a, 15b im Garn 1 darstellen. Ferner ist eine erste Reinigungsgrenze 14 eingezeichnet, die hier aus waagrechten und senkrechten Abschnitten treppenförmig zusammengesetzt ist. Fehler 15a, die unterhalb der ersten Reinigungsgrenze 14 liegen, gelten als zulässig und werden im Garn 1 belassen. Fehler 15b hingegen, die oberhalb der ersten Reinigungsgrenze 14 liegen, gelten als unzulässig. Ihr Auftreten löst eine Handlung aus, üblicherweise eine Entfernung des Fehlers 15b aus dem Garn 1 durch Herausschneiden des fehlerbehafteten Garnabschnitts.

In **Figur 4(b)** ist eine zweite Reinigungskurve 16 für Spleisse 3 eingezeichnet, die hier als durch ihre Koordinaten 6, 7 definierte Punkte 17a, 17b erscheinen. Die zweite Reinigungskurve 16 stellt eine Reinigungsgrenze für die Spleisspunkte oder Spleissereignisse 17a, 17b dar. Für die Spleisspunkte 17a, 17b definiert die zweite Reinigungsgrenze 16 ein Zulässigkeitskriterium, analog zur ersten Reinigungsgrenze 14. Spleisspunkte 17a, die unterhalb der zweiten Reinigungsgrenze 16 liegen, gelten als zulässig und werden im Garn 1 belassen. Spleisspunkte 17b hingegen, die oberhalb der zweiten Reinigungsgrenze 16 liegen, gelten als unzulässig. Ihr Auftreten löst eine Handlung aus, üblicherweise eine Entfernung des einem Spleisspunkt 17b entsprechenden Spleisses 3 aus dem Garn 1. Die zweite Reinigungskurve 16 kann z. B. durch Stauchung der ersten Reinigungskurve 14 in Richtung der Ordinate 7, z. B. mit dem Skalierungsfaktor 0.75, erhalten werden.

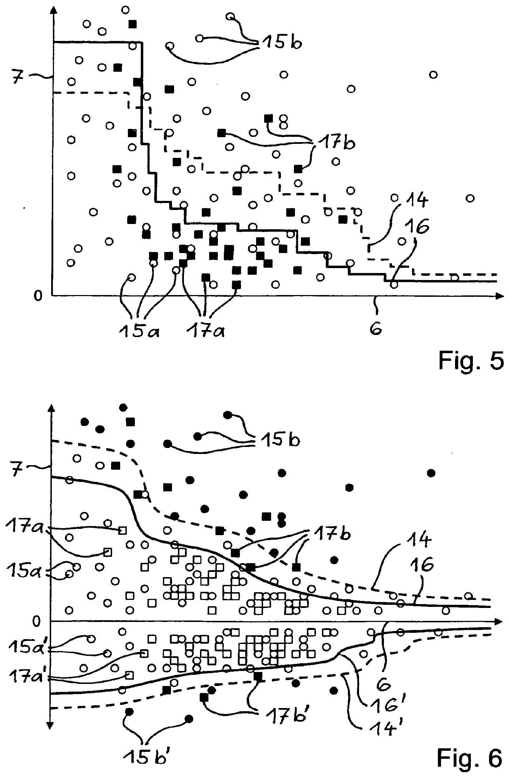

Während in Figur 4 die Fehlstellenereignisse 15a, 15b und die Spleissereignisse 17a, 17b in zwei voneinander getrennten Diagrammen dargestellt sind, vereinigt die **Figur 5** beide in einem einzigen Diagramm. Auch die erste und zweite Reinigungskurve 14, 16 sind beide in dem Diagramm eingezeichnet. Die Interpretation des Diagramms kann einer Bedienungsperson leichter fallen, wenn sich die Ereignispunkte 15a, 15b für Fehlstellen 2 von den Ereignispunkten 16a, 16b für Spleisse 3 graphisch unterscheiden. Eine solche Unterscheidung wurde im Beispiel von Figur 5 vorgenommen, indem die Ereignispunkte 15a, 15b für Fehlstellen 2 durch weiss gefüllte Kreise und die Ereignispunkte 17a, 17b für Spleisse 3 durch schwarz gefüllte Quadrate dargestellt wurden. Selbstverständlich können weitere Formen, Farben und/oder Schattierungen verwendet werden.

Ebenso fördern graphische Unterschiede in den Darstellungen der ersten Reinigungskurve 14 und der zweiten Reinigungskurve 16 die Übersichtlichkeit. So wurde im Beispiel von Figur 5 die erste Reinigungskurve 14 strichliert, die zweite Reinigungskurve 16 dagegen durchgehend eingezeichnet. Auch hier kommen weitere Symbole und/oder Farben in Frage.

Da typische Spleisslängen 4 zwischen ca. 2 und 10 cm liegen, könnte es genügen, die zweite Reinigungsgrenze 16 nur in diesem Längenintervall zu definieren; im Beispiel von Figur 5 ist sie dennoch für die ganze Längenachse 6 definiert. Die zweite Reinigungskurve 16 liegt vorzugsweise unterhalb der ersten Reinigungskurve 14. Dadurch wird sichergestellt, dass eine unzulässige Dickstelle 15b nicht etwa durch einen zulässigen Spleiss 2 ersetzt wird, der möglicherweise dicker und/oder länger ist als die entfernte Dickstelle 15b. Es kann aber Anwendungsfälle geben, in denen die beiden Reinigungskurven 14, 16 zusammenfallen oder in denen sogar die zweite Reinigungskurve 16 oberhalb der ersten Reinigungskurve 14 liegt. Im Beispiel von Figur 5 trifft Letzteres für kleine Längen in der Nähe der Ordinate 7 zu.

Die zweite Reinigungsgrenze 16 kann frei von einer Bedienungsperson festgelegt und/oder automatisch berechnet werden. Die manuelle Festlegung erfolgt vorzugsweise mit Hilfe einer Eingabeeinheit 37 und einer Ausgabeeinheit 38 (siehe Figur 5). Damit kann die zweite Reinigungskurve 16 gezeichnet und/oder nachträglich bearbeitet werden, ähnlich wie dies mit einfachen Zeichnungsprogrammen auf einem Personalcomputer möglich ist. Es ist möglich, einen Satz von vordefinierten Kurvenverläufen zur Verfügung zu stellen, von denen einer ausgewählt, in die gewünschte Position gebracht und bei Bedarf bearbeitet werden kann, ähnlich wie es die EP-1'295'835 A2 für Fehlstellen beschreibt.

Die Reinigungskurven 14, 16 verlaufen im Ausführungsbeispiel von Figur 5 stufenförmig und parallel zu den in Figur 2 eingezeichneten Klassengrenzen 11a-11d, 12a-12d; dies ist jedoch nicht notwendig. Es sind stetige Reinigungskurven 14, 16 oder Reinigungskurven 14, 16 mit stetigen und unstetigen Abschnitten möglich. **Figur 6** zeigt ein Diagramm entsprechend Figur 5, aber mit beliebigen, d. h. nicht an Klassengrenzen gebundenen Verläufen einer ersten Reinigungskurve 14 für Fehlstellen 2, die hier als Punkte 15a, 15b erscheinen, und einer zweiten Reinigungskurve 16 für Spleisse 3, die hier als Punkte 17a, 17b erscheinen. Die Ereignispunkte 15a für zulässige Fehlstellen 2 sind im Beispiel von Figur 6 als weiss gefüllte Kreise, die Ereignispunkte 15b für unzulässige Fehlstellen 2 als schwarz gefüllte Kreise dargestellt. Die Ereignispunkte 17a für zulässige Spleisse 3 sind im Beispiel von Figur 6 als weiss gefüllte Quadrate, die Ereignispunkte 17b für unzulässige Spleisse 3 als schwarz gefüllte Quadrate dargestellt. Somit unterscheidet diese Darstellung graphisch nicht nur zwischen Ereignispunkten 15a, 15b für Fehlstellen 2 einerseits und Ereignispunkten 17a, 17b für Spleisse 3 andererseits, sondern auch zwischen Ereignispunkten 15a, 17a für zulässige Ereignisse einerseits und Ereignispunkten 15b, 17b für unzulässige Ereignisse andererseits. Dies kann die Übersichtlichkeit zusätzlich erhöhen. Auch bezüglich dieser Unterscheidung stehen dem Fachmann viele graphische Merkmale und Mittel zur Verfügung.

Nebst Dickstellen können in einem Garn 1 auch Dünnstellen auftreten. Diese können sowohl durch den Garnherstellungsprozess als auch durch einen Spleissprozess verursacht sein. Im Diagramm von Figur 6 ist für die Darstellung der Dünnstellen ein eigener Quadrant unterhalb der Abszisse 6 vorgesehen, während die oben diskutierten Dickstellen in einem Quadranten oberhalb der Abszisse 6 eingezeichnet sind. Die herstellungsbedingten Dünnstellen sind mit kreisförmigen Punkten und den Bezugszeichen 15a', 15b' gekennzeichnet, die spleissbedingten Dünnstellen mit quadratischen Punkten und den Bezugszeichen 17a', 17b'. Entsprechende Reinigungskurven 14', 16' für die Dünnstellen sind ebenfalls eingezeichnet. Bezüglich der Zulässigkeit bzw. Unzulässigkeit der Dünnstellen gilt analog das für die Dickstellen Gesagte.

Um in einem beispielsweise textilen Garn 1 (vgl. Figur 1) die Qualität der Spleisse 3 zu verbessern, werden Parameter der Spleisse 3 wie Länge 4 und Durchmesser 5 oder Masse pro Längeneinheit (oder Änderungen des Durchmessers 3 bzw. der Masse pro Längeneinheit) gemessen und in einem Diagramm, wie es in den Figuren 2-6 dargestellt ist, als Punktewolke aufgetragen. So wird die Möglichkeit geschaffen, zu erkennen, ob ein bestimmter Spleiss 3 im Garn 1 stören würde oder nicht. Die Unterscheidung zwischen zulässigen und unzulässigen Spleissen 3 wird anhand der zweiten Reinigungsgrenze 16, 16' getroffen.

**Figur 7** zeigt eine Vorrichtung zur Durchführung des Verfahrens, wie sie z. B. an der Spulstelle einer Textilmaschine zum Einsatz kommen kann. Darin erkennt man einen Kops 23, von dem Garn 1 abgewickelt und auf eine Spule 24 aufgewickelt wird. Wenn wir davon ausgehen, dass sich das Garn 1 in Richtung eines Pfeils 25 bewegt, so findet man eine an sich bekannte erste Messeinrichtung 26 zum Messen von Parametern 4, 5 am Garn 1, eine zweite Messeinrichtung 27 zum Messen einer Momentangeschwindigkeit v des Garns 1, eine Schneideinheit 28 zum Entfernen von unzulässigen Fehlstellen aus dem Garn 1 und eine Spleisseinheit 29 zum Verbinden zweier Garnenden. Diese vier Einrichtungen 26-29 sind an sich bekannt und deshalb hier nicht näher beschrieben. Die zweite Messeinrichtung 27 für die Geschwindigkeit v des Garns 1 kann auch in die betreffende Spinn- oder Spulmaschine integriert sein, so dass in diesem Falle die Spinn- oder Spulmaschine ein Geschwindigkeitssignal liefert. Eine Auswerteeinheit 30 ist über Leitungen 31 bzw. 32 mit den beiden Messeinrichtungen 26 und 27 verbunden. Sie berechnet insbesondere die Länge 5 eines Spleisses 3 aus der in der ersten Messeinrichtung 26 gemessenen Zeitdauer des Durchgangs des Spleisses 3 und der in der zweiten Messeinrichtung 27 gemessenen Geschwindigkeit v des Garns 1. Die Auswerteeinheit 30 ist über eine Leitung 33 mit der Schneideinheit 28 verbunden, der sie Befehle zum Trennen des Garns 1 erteilen kann. Die erste Messeinrichtung 26, die Schneideinheit 28, die Auswerteeinheit 30 sowie eventuell auch die zweite Messeinrichtung 27 können in einem elektronischen Messkopf 34 eines elektronischen Garnreinigers integriert sein.

Ein Steuergerät 35 ist über eine Leitung 36 mit der Auswerteeinheit 30 verbunden. Das Steuergerät 35 steuert und informiert die Auswerteeinheit 30, z. B. indem es die erste und zweite Reinigungsgrenze an sie übermittelt. Von der Auswerteeinheit 30 empfängt es verschiedene Daten sowie Informationen und verarbeitet sie auch. Dazu gehören diejenigen Informationen, die es braucht, um die Spleisseinheit 29 falls nötig zu aktivieren; ferner können Daten zur Garnqualität und/oder Spleissqualität darunter sein. Vorzugsweise ist eine Eingabeeinheit 37, z. B. eine Tastatur, eine Computermaus oder dergleichen, und/oder eine Ausgabeeinheit 38, z. B. ein Bildschirm, zur Ein- bzw. Ausgabe von Daten am Steuergerät 35 angeschlossen oder in ihm integriert. Die Eingabeeinheit 37 und die Ausgabeeinheit 38 können in einem Sensorbildschirm (touchscreen) zusammengefasst sein. Das Steuergerät 35 ist vorzugsweise mit mehreren elektronischen Messköpfen 34 verbunden. Es kommuniziert über eine Leitung 40 auch mit einer Maschinensteuereinheit 39 der Textilmaschine. Die Maschinensteuereinheit ist wiederum über eine Leitung 42 mit einem Arbeitsstellenrechner oder Produktionsstellenrechner 41 verbunden, der seinerseits über Leitungen 43 bzw. 44 mit der Spleisseinheit 29 und der Auswerteeinheit 30 verbunden ist.

Die Darstellung der Parameter 4, 5 von erzeugten Spleissen 3 erlaubt es, Einstellungen an der Spulmaschine oder der Spleisseinheit 29 zu verändern, wenn man feststellt, dass die Parameterwerte 4, 5 stark streuen oder aus einem anderen Grund inakzeptabel sind. In solchen Fällen kann eine Einstellung der Spleisseinheit 29 geändert werden, beispielsweise die Zufuhr von Druckluft, die Auflösung der Garndrehung, die Verwirbelung der beiden Garnenden, beim Thermospleissen die Zufuhr von Wärmeenergie oder beim Nassspleissen der Eintrag von Flüssigkeit. Für die Spleisse 3 können auch Fehlerklassen bestimmt werden, wie dies die Figur 2 zeigt. Ein Vergleich der Klassen, in denen die Spleisse 3 liegen, mit Klassen, in denen Garnfehler 2 liegen, erlaubt es, eine begründete Beurteilung über die Tolerierbarkeit der erfassten Spleisse 3 vorzunehmen. Dazu wird die Anzahl Fehler in benachbarten Klassen und ein Abstand zwischen jenen Klassen, in denen die häufigsten Fehler 2 im Garn 1 liegen, und der Klasse, in der die Spleisse 3 liegen, in die Beurteilung einfliessen.

Es ist zu bedenken, dass die Textilmaschine üblicherweise zur Erzeugung eines Spleisses 3, z. B. nach dem Herausschneiden einer Fehlstelle 2 oder bei einem Spulenwechsel, für eine kurze Zeit stillgesetzt wird und danach wieder hochgefahren wird. **Figur 8** zeigt schematisch einen möglichen Verlauf 63 der Geschwindigkeit v eines Garns 1 während des Anfahrens der Textilmaschine, wobei Werte für die Garngeschwindigkeit v längs einer vertikalen Achse 61 über einer Zeitachse 60 aufgetragen sind. Bei still stehendem Garn 1 werden die beiden Garnenden über einen Spleiss 3 zusammengefügt. Danach wird die Garngeschwindigkeit v von null auf eine Endgeschwindigkeit 62 erhöht. Die Messung der Spleissparameter 4, 5 erfolgt in einem Zeitabschnitt 65, wie er in der Figur 8 durch vertikale Geraden 66 und 67 eingegrenzt ist. In diesem Zeitabschnitt 65 ist die Geschwindigkeit v des Garns 1 nicht konstant, sondern verändert sich mit der Zeit. Während der Beschleunigung des Garns 1 muss auch der Spleiss 3 gemäss der vorliegenden Erfindung überwacht werden. Die sich zeitlich verändernde Geschwindigkeit v ist eine Erschwernis bei der Messung der Parameterwerte 4, 5 am erstellten Spleiss 3. Um in diesem Falle bspw. eine zuverlässige Messung der Länge 5 eines Spleisses 3 zu erhalten, muss die augenblickliche Geschwindigkeit v des Garns 1 in die Verarbeitung der Messwerte einfliessen. Dazu ist im Verfahren ein Geschwindigkeitssignal aus der zweiten Messeinrichtung 27 (vgl. Figur 5) notwendig, das dazu verwendet wird, die Zeit, welche die erste Messeinrichtung 26 zwischen dem Anfang und dem Ende eines Spleisses 3 misst, in eine Spleisslänge 5 umzurechnen.

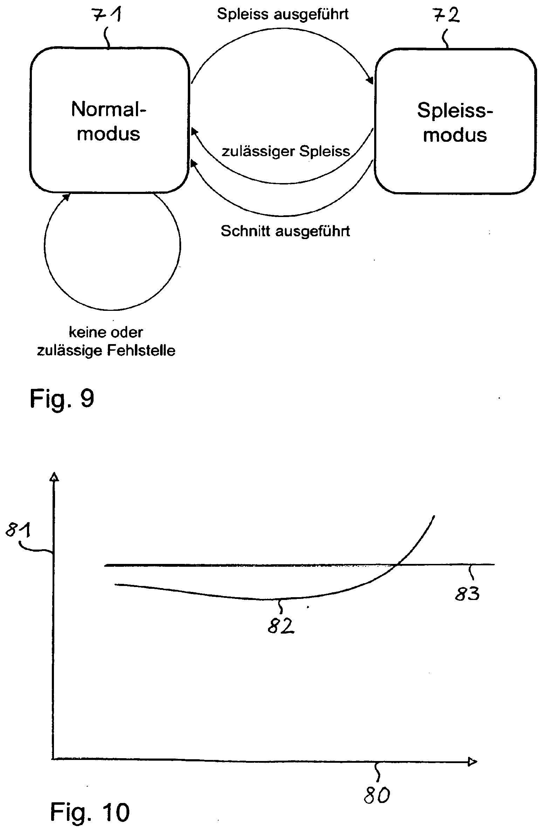

Dementsprechend soll die Vorrichtung in zwei verschiedenen Arbeitsmodi betrieben werden können: einem ersten für die bekannte Messung von Parametern 4, 5 von Fehlern 2 im Garn 1, die während des normalen Arbeitsprozesses erfolgt, und einem zweiten für die Messung von Parametern 4, 5 an Spleissen 3, die während des Anfahrens der Textilmaschine erfolgt. **Figur 9** zeigt ein Zustandsdiagramm mit den beiden Arbeitsmodi 71, 72. Der erste Arbeitsmodus 71, hier Normalmodus genannt, ist der aus dem Stand der Technik bekannte Normalfall, in dem das Garn 1 unter Verwendung der ersten Reinigungsgrenze 14 auf mögliche Fehlstellen 2 hin überwacht wird. Der zweite Arbeitsmodus 72, hier Spleissmodus genannt, dient der Überwachung von Spleissen 3 unter Verwendung der zweiten Reinigungsgrenze 16. Die Umstellung von einem Arbeitsmodus auf den anderen kann durch die Auswerteeinheit 30 (vgl. Figur 5) erfolgen. Wenn z. B. die Auswerteeinheit 30 von der zweiten Messeinrichtung 27 die Information erhält, dass das Garn 1 still steht, oder vom Arbeitsstellenrechner 41 die Information erhält, dass ein Spleiss 3 durch die Spleisseinheit 29 erzeugt wurde, stellt sie auf den Spleissmodus 72 um. Sobald der erzeugte Spleiss 3 für zulässig befunden wurde oder der Spleiss 3 für unzulässig befunden und herausgeschnitten wurde, stellt die Auswerteeinheit 30 auf den Normalmodus 71 um.

Nach erfolgter Messung von Werten für mindestens einen Parameter 4, 5 von Spleissen 3 ist es auch wünschbar, die innerhalb einer bestimmten Garnlänge oder Zeitdauer festgestellten Spleisse 3 zu zählen. Die Spleisse 3 werden je nach gemessenen Werten zwei Gruppen zugeordnet, und die Ereignisse (Spleisse 3) in jeder Gruppe werden gezählt. Dabei soll eine Anzahl $J_u$ unzulässiger Spleisse 17b in einer ersten Gruppe gezählt werden und die gesamte Anzahl $J_{tot}$ der Spleisse 3 in einer zweiten Gruppe ebenfalls gezählt werden. Bildet man aus den Zählwerten $J_u$, $J_{tot}$ der beiden Gruppen laufend ein Spleissverhältnis $Q = J_u/J_{tot}$, so kann man daraus ableiten, ob eine bestimmte Spleisseinheit 29 (vgl. Figur 5) gut oder schlecht arbeitet. Wenn z. B. das Spleissverhältnis Q unterhalb eines vorgegebenen Schwellwertes $Q_g$ liegt ($Q \leq Q_g$), funktioniert die Spleisseinheit 29 gut, andernfalls ($Q > Q_g$) schlecht. Im letzteren Fall kann ein Alarmsignal ausgelöst werden, das bewirkt, dass die Spleisseinheit 29 oder die Spulstelle, in der sie eingebaut ist, zur Überprüfung stillgesetzt wird.

**Figur 10** zeigt ein Diagramm mit einer Achse 81, längs der Werte eines Verhältnisses $J_u/J_{tot}$ der Anzahl $J_u$ von unzulässigen Spleissen 17b zu der gesamten Anzahl $J_{tot}$ aller erfassten Spleisse 3 aufgetragen sind. Zudem ist eine Zeitachse 80 vorgesehen. Darüber ist eine Kurve 82 aufgezeichnet, die den zeitlichen Verlauf des Spleissverhältnis $Q = J_u/J_{tot}$ darstellt. Ferner ist ein zeitlich konstanter Schwellwert $Q_g$ für das Spleissverhältnis Q als waagrechte Gerade 83 eingezeichnet. Im linken Bereich des Diagramms liegt die Kurve 82 unter dem Schwellwert $Q_g$ ($Q \leq Q_g$), was auf eine gut funktionierende 29 hindeutet. Hingegen überschreitet die Kurve 82 im rechten Bereich des Diagramms den Schwellwert $Q_g$ ($Q > Q_g$), so dass zum Zeitpunkt des Überschreitens ein Alarmsignal ausgegeben werden sollte.

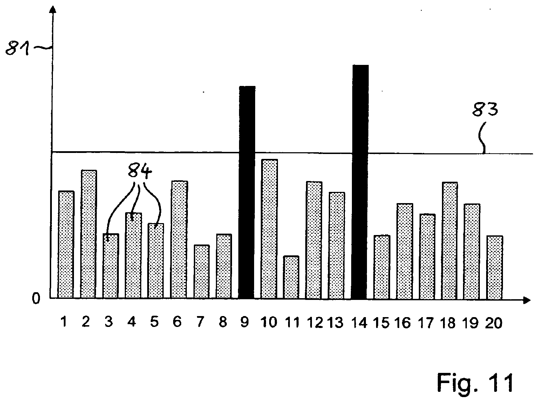

Im Diagramm von **Figur 11** sind die Spleissverhältnisse $Q = J_u/J_{tot}$ zu einem bestimmten Zeitpunkt für mehrere, z. B. 20, Spleisseinheiten 29 in Form von Säulen 84 dargestellt. Der Schwellwert $Q_g$ für das Spleissverhältnis Q ist wiederum als waagrechte Gerade 83 eingezeichnet. Eine solche graphische Darstellung ermöglicht einen schnellen, einfachen Überblick über das Funktionieren vieler Spleisseinheiten 29, z. B. auf einer automatischen Spulmaschine. So erkennt man sofort, dass im Beispiel von Figur 11 die Spleisseinheiten 29 an den Positionen Nr. 9 und Nr. 14 zu hohe, den Schwellwert $Q_g$ überschreitende Spleissverhältnisse Q aufweisen und somit einer Neueinstellung, einer Reparatur oder eines Ersatzes bedürfen. Auch hier kann beim Überschreiten des Schwellwertes $Q_g$ ein Alarmsignal ausgegeben werden. Die Höhe der Säulen 84 kann laufend oder periodisch aktualisiert werden, so dass das Diagramm eine aktuelle Übersicht liefert.

BEZUGSZEICHENLISTE

| | |
|---|---|
| 1 | Garn |
| 2 | Fehlstelle |
| 3 | Spleiss |
| 4 | Fehlerlänge oder Spleisslänge |
| 5 | Parameter Durchmesser oder Masse pro Längeneinheit |
| 6 | Abszisse, Längenachse |
| 7 | Ordinate, Durchmesser- oder Massenachse |
| 8 | Spleisspunkte |
| 9, 10 | Geraden, zur Angabe der Koordinaten eines Spleisspunktes |
| 11, 12 | Klassengrenzen |
| 13 | Punktewolke |
| 14 | Reinigungsgrenze bzw. Reinigungskurve für Fehlstellen 2 |
| 15a | zulässige Fehlstellenereignisse |
| 15b | unzulässige Fehlstellenereignisse |
| 16 | Reinigungsgrenze bzw. Reinigungskurve für Spleisse 3 |
| 17a | zulässige Spleissereignisse |
| 17b | unzulässige Spleissereignisse |
| 23 | Kops |
| 24 | Spule |
| 25 | Bewegungsrichtung des Garns 1 |
| 26 | Messeinrichtung für Garnparameter |
| 27 | Messeinrichtung für Garngeschwindigkeit |
| 28 | Schneideinheit |
| 29 | Spleisseinheit |

| 30 | Auswerteinheit |
|---|---|
| 31-33, 36, 40, 42-44 | Leitungen |
| 34 | elektronischer Messkopf |
| 35 | Steuergerät |
| 37 | Eingabeeinheit |
| 38 | Ausgabeeinheit |
| 39 | Maschinensteuereinheit |
| 41 | Arbeitsstellenrechner oder Produktionsstellenrechner |
| 60 | Zeitachse |
| 61 | Geschwindigkeitsachse |
| 62 | Endgeschwindigkeit |
| 63 | zeitlicher Verlauf der Geschwindigkeit |
| 65 | Zeitintervall, während dessen Spleiss 3 erzeugt wird |
| 66 | Beginn des Spleissvorgangs |
| 67 | Ende des Spleissvorgangs |
| 71 | Normalmodus |
| 72 | Spleissmodus |
| 80 | Zeitachse |
| 81 | Spleissverhältnis |
| 82 | zeitlicher Verlauf des Spleissverhältnisses |
| 83 | Schwellwert für das Spleissverhältnis |
| 84 | Säulen, die das Spleissverhältnis darstellen |

**Patentansprüche**

1. Vorrichtung zur Überwachung von Spleissen (3) in einem länglichen textilen Prüfgut (1), mit einem elektronischen Messkopf (34), durch welchen das Prüfgut (1) in seiner Längsrichtung (25) bewegbar ist, zur Messung mindestens eines Parameters (4, 5) des Prüfgutes (1) und

   einer Steuereinheit (35) zum Einteilen von Spleissen (3) aufgrund mindestens eines gemessenen Parameters (4, 5) in zulässige (17a) bzw. unzulässige (17b) Spleisse (3), **dadurch gekennzeichnet, dass**

   die Steuereinheit (35) zum Verknüpfen von mindestens zwei der drei Grössen aus der Menge, die aus

   der Anzahl ($J_u$) der unzulässigen Spleisse (17b),

   der Anzahl ($J_z$) der zulässigen Spleisse (17a) und

   der Anzahl ($J_{tot}$) der insgesamt erfassten Spleisse

   besteht, zu einer einzigen, die Spleissqualität charakterisierenden Spleissqualitätsgrösse (Q) eingerichtet ist,

   die Steuereinheit (35)

   zur Bildung eines Verhältnisses als Verknüpfung eingerichtet ist, insbesondere eines Verhältnisses (Q) zwischen der Anzahl ($J_u$) der unzulässigen Spleisse (17b) oder der Anzahl ($J_z$) zulässigen Spleisse (17a) einerseits und der Gesamtzahl ($J_{tot}$) der erfassten Spleisse andererseits, und

   die Steuereinheit (35) zur Auslösung einer Handlung, bspw. zur Ausgabe eines Alarmsignals, wenn die Spleissqualitätsgrösse (Q) einen vorgegebenen Schwellwert ($Q_g$) über- bzw. unterschreitet, eingerichtet ist.

2. Vorrichtung nach Anspruch 1, wobei

   der elektronische Messkopf (34) zur Messung von mindestens jeweils zwei Parametern (4, 5) des Prüfgutes (1) für Fehlstellen (2) und für Spleisse (3) im Prüfgut (1) eingerichtet ist,

   die Steuereinheit (35) dazu eingerichtet ist,

   eine von den mindestens zwei Parametern (4, 5) abhängige erste Reinigungsgrenze zwischen zulässigen (15a) und unzulässigen (15b) Fehlstellen (2) im Prüfgut (1) zu speichern,

   die erste Reinigungsgrenze an den Messkopf (34) zu übermitteln sowie eine von den mindestens zwei Parametern (4, 5) abhängige, von der ersten Reinigungsgrenze verschiedene zweite Reinigungsgrenze zwischen zulässigen (17a) und unzulässigen (17b) Spleissen (3) im Prüfgut (1) aus der ersten Reinigungsgrenze automatisch zu berechnen, zu speichern und an den Messkopf (34) zu übermitteln, und

   eine mit der Steuereinheit (35) verbundene Anzeigeeinheit (38) dazu eingerichtet ist,

   die erste Reinigungsgrenze als erste Reinigungskurve (14) in einem Diagramm darzustellen sowie

   die zweite Reinigungsgrenze als zweite Reinigungskurve (16) in einem Diagramm darzustellen.

3. Vorrichtung nach Anspruch 2, wobei die Vorrichtung eine mit der Auswerteinheit (30) verbundene Schneideinheit

(27) zum Entfernen von unzulässigen Spleissen aus dem Prüfgut (1) beinhaltet.

4. Vorrichtung nach Anspruch 2 oder 3, wobei
die Steuereinheit (35) dazu eingerichtet ist,
die mindestens zwei Parameter (4, 5) des Prüfgutes (1) für Fehlstellen (2) im Prüfgut (1) mit der ersten Reinigungsgrenze zu vergleichen,
sowie
die mindestens zwei Parameter (4, 5) des Prüfgutes (1) für Spleisse (3) im Prüfgut (1) mit der zweiten Reinigungsgrenze zu vergleichen, und
die Anzeigeeinheit (38) dazu eingerichtet ist,
die mindestens zwei Parameter (4, 5) des Prüfgutes (1) für Fehlstellen (2) im Prüfgut (1) in Form einer ersten Punktewolke (13), in der jeder Punkt (15a, 15b) einer Fehlstelle (2) entspricht, in dem Diagramm darzustellen sowie
die mindestens zwei Parameter (4, 5) des Prüfgutes (1) für Spleisse (3) im Prüfgut (1) in Form einer Punktewolke (13), in der jeder Punkt (17a, 17b) einem Spleiss (3) entspricht, in dem Diagramm darzustellen.

**Claims**

1. A device for monitoring splices (3) in an elongate textile product to be tested (1), with an electronic measurement head (34), through which the product to be tested (1) is movable in its longitudinal direction (25), for measuring at least one parameter (4, 5) of the product to be tested (1), and
a control unit (35) for dividing splices (3) into allowable (17a) or unallowable (17b) splices (3) on account of at least one measured parameter (4, 5),
**characterised in that**
the control unit (35) is set up to combine at least two of the three variables from the set which consists of
the number ($J_u$) of the unallowable splices (17b),
the number ($J_z$) of the allowable splices (17a) and
the number ($J_{tot}$) of the splices detected in total,
into a single splice quality variable (Q) which characterizes the splice quality,
the control unit (35) is set up for forming a ratio as a combination, in particular a ratio (Q) between the number ($J_u$) of the unallowable splices (17b) or the number ($J_z$) of the allowable splices (17a) on the one hand, and the total number ($J_{tot}$) of the detected splices on the other hand, and
the control unit (35) is set up for triggering an action, for example for issuing an alarm, if the splice quality variable (Q) exceeds or falls short of a predefined threshold value ($Q_E$).

2. The device according to claim 1, wherein
the electronic measurement head (34) is set up for measuring at least in each case two parameters (4, 5) of the product to be tested (1) for defects (2) and for splices (3) in the product to be tested (1),
the control unit (35) is set up
for storing a first clearing limit between allowable (15a) and unallowable (15b) defects (2) in the product to be tested (1), which first clearing limit is dependent on the at least two parameters (4, 5),
for transmitting the first clearing limit to the measurement head (34) and
for automatically computing from the first clearing limit a second clearing limit between allowable (17a) and unallowable (17b) splices (3) in the product to be tested (1), which second clearing limit is dependent on the at least two parameters (4, 5) and is different from the first clearing limit, for storing it and for transmitting it to the measurement head (34), and
a display unit (38) connected to the control unit (35) is set up
for representing the first clearing limit as a first clearing curve (14) in a diagram and
for representing the second clearing limit as a second clearing curve (16) in a diagram.

3. The device according to claim 2, wherein the device contains a cutting unit (27) connected to the evaluation unit (30), for removing unallowable splices from the product to be tested (1).

4. The device according to claim 2 or 3, wherein
the control unit (35) is set up
for comparing the at least two parameters (4, 5) of the product to be tested (1) for defects (2) in the product to be tested (1) to the first clearing limit and
for comparing the at least two parameters (4, 5) of the product to be tested (1) for splices (3) in the product to be tested (1) to the second clearing limit, and

the display unit (38) is set up

for representing the at least two parameters (4, 5) of the product to be tested (1) for defect locations in the product to be tested (1) in the diagram in the form of a first scatter plot (13), in which each point (15a, 15b) corresponds to a defect location (2), and

for representing the at least two parameters (4, 5) of the product to be tested (1) for splices (3) in the product to be tested (1) in the diagram in the form of a scatter plot (13), in which each point (17a, 17b) corresponds to a splice (3).


**Revendications**

1. Dispositif pour la surveillance d'épissures (3) dans une matière textile à contrôler (1) allongée, avec

   une tête de mesure électronique (34) à travers laquelle la matière à contrôler (1) peut être déplacée dans le sens de sa longueur (25), pour la mesure d'au moins un paramètre (4, 5) de la matière à contrôler (1), et

   une unité de commande (35) pour classer les épissures (3) en fonction d'au moins un paramètre mesuré (4, 5) en épissures (3) admissibles (17a) et non admissibles (17b),

   **caractérisé**

   **en ce que** l'unité de commande (35) est équipée pour associer au moins deux des trois grandeurs parmi le groupe composé

   du nombre ($J_u$) des épissures non admissibles (17b),

   du nombre ($J_z$) des épissures admissibles (17a) et

   du nombre ($J_{tot}$) des épissures détectées au total

   pour donner une seule grandeur de qualité des épissures (Q) qui caractérise la qualité des épissures,

   **en ce que** l'unité de commande (35) est équipée pour calculer un rapport sous la forme d'une association, en particulier un rapport (Q) entre le nombre ($J_u$) des épissures non admissibles (17b) ou le nombre ($J_z$) des épissures admissibles (17a) d'une part et le nombre total ($J_{tot}$) des épissures détectées d'autre part, et

   **en ce que** l'unité de commande (35) est équipée pour déclencher une action, en particulier pour émettre un signal d'alarme, quand la grandeur de qualité des épissures (Q) est supérieure ou inférieure à une valeur de seuil (Qg) prédéterminée.

2. Dispositif selon la revendication 1, dans lequel

   la tête de mesure électronique (34) est équipée pour mesurer au moins deux paramètres (4, 5) de la matière à contrôler (1) pour les défauts (2) et pour les épissures (3) de la matière à contrôler (1),

   l'unité de commande (35) est équipée pour

   enregistrer une première limite de nettoyage dépendante des au moins deux paramètres (4, 5) entre des défauts (2) admissibles (15a) et non admissibles (15b) de la matière à contrôler (1),

   transmettre la première limite de nettoyage à la tête de mesure (34) et

   calculer automatiquement une deuxième limite de nettoyage dépendante des au moins deux paramètres (4, 5) et différente de la première limite de nettoyage entre les épissures (3) admissibles (17a) et non admissibles (17b) de la matière à contrôler (1) à partir de la première limite de nettoyage, l'enregistrer et la transmettre à la tête de mesure (34), et

   une unité d'affichage (38) reliée à l'unité de commande (35) est équipée pour

   représenter la première limite de nettoyage sous la forme d'une première courbe de nettoyage (14) dans un diagramme et

   représenter la deuxième limite de nettoyage sous la forme d'une deuxième courbe de nettoyage (16) dans un diagramme.

3. Dispositif selon la revendication 2, dans lequel le dispositif présente une unité de coupe (27) reliée à l'unité d'analyse (30) pour éliminer les épissures non admissibles de la matière à contrôler (1).

4. Dispositif selon l'une des revendications 2 ou 3, dans lequel l'unité de commande (35) est équipée pour

   comparer les au moins deux paramètres (4, 5) de la matière à contrôler (1) pour les défauts (2) de la matière à contrôler (1) avec la première limite de nettoyage et comparer les au moins deux paramètres (4, 5) de la matière à contrôler (1) pour les épissures (3) de la matière à contrôler (1) avec la deuxième limite de nettoyage, et l'unité d'affichage (38) est équipée pour

   représenter dans le diagramme les au moins deux paramètres (4, 5) de la matière à contrôler (1) pour les défauts (2) de la matière à contrôler (1) sous la forme d'un premier nuage de points (13) dans lequel chaque point (15a, 15b) correspond à un défaut (2) et

représenter dans le diagramme les au moins deux paramètres (4, 5) de la matière à contrôler (1) pour les épissures (3) de la matière à contrôler (1) sous la forme d'un nuage de points (13) dans lequel chaque point (17a, 17b) correspond à une épissure (3).

Fig. 1(a)

Fig. 1(b)

Fig. 7

**Fig. 2**

**Fig. 8**

Fig. 3(a)

Fig. 3(b)

Fig. 3(c)

Fig. 4(a)

Fig. 4(b)

Fig. 5

Fig. 6

Fig. 9

Fig. 10

Fig. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4028465 A1
- DE 3937824 A1
- DE 19649329 A1
- EP 1101846 A2
- EP 1077194 A2
- EP 1295835 A2
- US 6374152 B1
- EP 1249422 A2
- WO 2007056883 A2